# EUROPÄISCHE PATENTSCHRIFT

(11) **EP 1 287 819 B1**
(45) Veröffentlichungstag und Bekanntmachung des Hinweises auf die Patenterteilung: **26.03.2008**
(21) Anmeldenummer: 02018323.2
(22) Anmeldetag: 14.08.2002
(51) Int. Cl.: A61K 9/00

(54) **Verfahren zur antibiotischen Beschichtung von Körpern mit interkonnektierenden Mikrohohlräumen sowie so beschichtete Körper und deren Verwendung**
Method of coating with antibiotics an object having interconnected microcavities and uses thereof
Méthode de recouvrement avec antibiotiques d'un objet à micropores interconnectés et leurs utilisations

(30) Priorität: 31.08.2001 DE 10142464; 01.02.2002 DE 10204308
(43) Veröffentlichungstag der Anmeldung: 05.03.2003
(73) Patentinhaber: Heraeus Kulzer GmbH, 63450 Hanau (DE)
(72) Erfinder: Vogt, Sebastian F., Dr., 07749 Jena (DE); Schnabelrauch, Matthias, Dr., 07749 Jena (DE); Kühn, Klaus-Dieter, Dr., 35041 Marburg (DE)
(74) Vertreter: Kühn, Hans-Christian

(56) Entgegenhaltungen:
- EP-A- 0 287 488
- EP-A- 0 414 914
- EP-A- 0 464 040
- EP-A- 0 467 954
- EP-A- 0 528 846
- EP-A- 0 535 937
- EP-A- 0 580 428
- EP-A- 0 719 845
- EP-A- 0 764 169
- US-A- 4 322 398
- US-A- 4 879 135
- US-A- 5 679 646
- "Römpp Chemie Lexikon" StartDateMarker 1995, EndDateMarker * Seite 2537 - Seite 2541 *

## Beschreibung

Die vorliegende Erfindung betrifft ein Verfahren zur antibiotischen Beschichtung von Körpern mit interkonnektierenden Mikrohohlräumen durch Behandlung mit einem 2-Komponenten-System.

Knochendefekte treten in der Human- und Veterinärmedizin relativ häufig auf und werden insbesondere durch Knochenfisteln, Trümmerfrakturen und Tumoren verursacht. Bei offenen Trümmerfrakturen werden vielfach zusätzlich Infektionen des Knochengewebes beobachtet. Die Behandlung von Knochendefekten kann durch Auffüllung mit geeigneten Implantaten erfolgen. In den letzten Jahren haben insbesondere poröse Implantate Interesse gefunden, die aufgrund ihrer chemischen Zusammensetzung und ihrer porösen Struktur eine osteokonduktive Wirkung aufweisen und ein Einwachsen des umgebenden Knochengewebes begünstigen. Problematisch ist die Behandlung von Knochendefekten immer dann, wenn zusätzlich mikrobielle Infektionen des Knochengewebes vorhanden sind. Infektionen des Knochengewebes können durch systemische oder lokale Applikation von geeigneten Antibiotika bekämpft werden. Die systemische Anwendung von Antibiotika ist aufgrund der mitunter nicht unbeträchtlichen Toxizität der Antibiotika problematisch. Die lokale Applikation direkt im oder am infizierten Gewebe bietet dagegen den Vorteil, dass hohe lokale Antibiotika-Konzentrationen erreicht werden können unter Vermeidung von schädigenden Antibiotika-Konzentrationen im übrigen Organismus. Durch diese hohen lokalen Antibiotika-Konzentrationen am Ort der bakteriellen Infektion ist eine fast vollständige Abtötung der Mikroorganismen möglich, so dass die bakteriellen Infektionen sehr wirksam behandelt werden. Besonders vorteilhaft ist es, wenn am Ort der bakteriellen Infektionen eine wirksame Antibiotikum-Konzentration über einen Zeitraum von mehreren Tagen bis Wochen aufrechterhalten wird, damit das Antibiotikum möglichst tief in das infizierte Gewebe eindringen kann und dadurch auch schwer zugängliche Keime vernichtet werden. Weichteildefekte mit bakteriellen Infektionen sind in der Human- und Veterinärmedizin ebenfalls häufig zu finden. Zur Behandlung dieser Infektionen ist daher auch die lokale Antibiotika-Behandlung von Interesse.

Bisher fanden schwerlösliche Salze der Aminoglykosid-Antibiotika, der Tetracyclin-Antibiotika und der Lincosamid-Antibiotika relativ geringe Beachtung für die Herstellung von Depotpräparaten und von antibiotisch wirksamen Implantaten. Die Synthese von schwerlöslichen Salzen bzw. Chelaten der Antibiotika des Tetracyclintyps sind seit Jahrzehnten allgemeiner Kenntnisstand. So beschreibt Folch Vazquez die Herstellung von Tetracyclindodecylsulfat durch Umsetzung von Tetracyclinhydrochlorid mit Natriumdodecylsulfat in Wasser (C. Folch-Vazquez: Tetracycline lauryl sulfate. 08.02.1966, ES 3309402; C. Folch Vazquez: Tetracycline derivatives. 09.01.1967, NL 6609490).

In US 4,879,135werden Oberflächen z.B. Kunsthoffoberflächen oder Metalloberflächen, mit Lösungen von anionischen Tensiden in Kontakt gebracht, wobei sich Tensidmoleküle an die Oberflächen anlagen. Nichtangelagerte Tensidreste werden gegebenenfalls danach abgewaschen. In einem zweiten Schritt werden die so vorbehandelten Oberflächen mit einem antithrombogenen Reagens oder einem kationischen Antibiotikum in Kontakt gebracht.

Bei den Aminoglykosid-Antibiotika sind ebenfalls eine Reihe von geringlöslichen Salzen prinzipiell bekannt. So wurde beim Gentamicin die Darstellung geringlöslicher Salze basierend auf höheren Fettsäuren, Arylalkylcarbonsäuren, Alkylsulfaten und Alkylsulfonaten beschrieben (G. M. Luedemann, M. J. Weinstein: Gentamycin and method of production. 16.07.1962, US 3,091,572). Exemplarisch sind dafür Gentamicin-Salze der Laurinsäure, der Stearinsäure, der Palmitinsäure, der Ölsäure, der Phenylbuttersäure, der Naphthalen-1-carbonsäure. Die Synthese der Dodecylsulfate des Gentamicins in wässriger bzw. wässrig-methanolischer Lösung sind von Jurado Soler et al. beschrieben worden (A. Jurado Soler, J. A. Ortiz Hernandez, C. Ciuro Bertran: Neue Gentamicinderivate, Verfahren zur Herstellung derselben und diese enthaltende antibiotisch wirksame Zusammensetzung. 30.09.1974, DE 24 46 640). Diese Salze erwiesen sich jedoch vielfach als unvorteilhaft, weil sie wachsartige, hydrophobe Substanzen darstellen, die eine galenische Verwendung behindern. Weiterhin wurden Fettsäuresalze und aliphatische Sulfate von Gentamicin und von Etamycin aus der freien Base bzw. aus ihren Salzen in Wasser bei 50 - 80°C synthetisiert (H. Voege, P. Stadler, H. J. Zeiler, S. Samaan, K.G. Metzger: Schwerlösliche Salze von Aminoglykosiden sowie diese enthaltende Formulierungen mit verzögerter Wirkstoff-Freigabe. 28.12.1982, DE 32 48 328). Diese Antibiotika-Fettsäuresalze sollen als Injektionspräparate geeignet sein. Eine neuere Entwicklung stellen schwerlösliche Aminoglykosid-Flavonoid-Phosphate dar (H. Wahlig, E. Dingeldein, R. Kirchlechner, D. Orth, W. Rogalski: Flavonoid phosphate salts of aminoglycoside antibiotics. 13.10.1986, US 4,617,293). Es werden die Salze der Phosphorsäuremonoester von Derivaten der Hydroxyflavane, Hydroxyflavene, Hydroxyflavanone, Hydroxyflavone und Hydroxyflavylium beschrieben. Besonders bevorzugt sind dabei die Derivate der Flavanone und der Flavone. Diese schwerlöslichen Salze sollen als Depotpräparate Verwendung finden. So werden zum Beispiel diese Salze in Kollagenvliese eingebracht (H. Wahlig, E. Dingeldein, D. Braun: Medicinally useful, shaped mass of collagen resorbable in the body. 22.09.1981, US 4,291,013). Weiterhin wurden auch künstliche Herzklappen mit diesen schwerlöslichen Gentamicin-Salzen, Gentamicin Crobefat, imprägniert (M. Cimbollek, B.Nies, R. Wenz, J. Kreuter: Antibiotic-impregnated heart valve sewing rings for treatment and prophylaxis of bacterial endocarditis. Antimicrob. Agents Chemother. 40(6) (1996) 1432-1437).

Die Erzeugung von einfachen Antibiotikum-/Antibiotka-Depots in den Porensystemen von porösen Körpern durch Tränken von porösen Körpern mit wässigen Antibiotika-Lösungen ist allgemeiner Kenntnisstand (R. Reiner, W. Kißing, H. Döring, K. Köster, H. Heide: Implantierbares Pharmaka-Depot. 20.02.1978, DE 28 07 132). Hierbei kann eine retardierende Wirkstofffreisetzung des in Wasser löslichen Wirkstoffs durch Adsorptions- und oder durch Diffusionsprozesse erreicht werden, die vom verwendeten Material, dem Porenvolumen und der Porosität abhängt.

Daneben ist es auch möglich, in Wasser gering lösliche Antibiotika-Salze in geeigneten organischen Lösungsmitteln zu lösen und mit diesen Lösungen die Formkörper zu tränken. Dadurch entstehen Wirkstoffdepots in den Formkörpern, die eine retardierende Wirkstofffreisetzung zeigen. Ein Beispiel dafür ist die von Cimbollek und Nies beschriebene Methode zur Lösung eines in Wasser gering löslichen Gentamicin-Salzes und deren Verwendung zur Beschichtung (M. Cimbollek, B. Nies: Solvent for a sparinly soluble gentamicin salt. 04.05.1994, US 5,679,646). Diese Gentamicinsalz auf Basis von 3-p-Methoxybezylidene-6-hydroxy-4'-methoxyflavanone-6-phosphat muss jedoch vor der Beschichtung synthetisiert werden. Von Kurtz wird eine sehr interessante Variante beschrieben, bei der in Wasser gering lösliche Antibiotika-Salze in situ auf einer saugfähigen Unterlage wie Verbandmaterial durch aufeinanderfolgende Tränkung mit einer Lösung eines basischen Gentamicin-Salzes bzw. eines Polymycin-Salzes und eines sauren Penicillin- bzw. Cephalosporinsalzes unter Ausfällung gebildet werden (L. D. Kurtz: Wasserunlösliche biocide Antibiotikasalze. 13.11.1973, DE 23 01 633). Die Penicillin- bzw. Cephalosporinreste bilden die anionische Komponente der Salze und die kationischen Aminoglukosid-Reste die kationische Komponente.

Dieses interessante Konzept wurde später nicht wieder aufgegriffen und auch nicht auf seine Eignung für andere in Wasser gering lösliche Salze der Aminoglykosid-Antibiotika, der Tetracyclin-Antibiotika, der Lincosamid-Antibiotika und der 4-Chinolon-Antibiotika geprüft. Bisher sind keine ähnlichen Imprägnierungsverfahren zur Erzeugung von Antibiotika-Depots in porösen Körpern unter Nutzung von anionischen Resten aus den Gruppen der organischen Sulfate und Sulfonate bekannt.

Die Schichtbildungseigenschaften von in Wasser gering löslichen Antibiotika-Salzen auf Basis von organischen Sulfaten und Sulfonaten fand ebenfalls bisher keine Beachtung.

Zusammenfassend kann festgestellt werden, dass bisher keine Verfahren bekannt sind, bei denen antibiotische Beschichtungen auf der Oberfläche von interkonnektierenden Porensystemen aufgebracht werden, die aus in Wasser gering löslichen Salzen der Aminoglukosid-Antibiotika, der Tetracyclin-Antibiotika, der Lincosamid-Antibiotika und der 4-Chinolon-Antibiotika bestehen und die direkt in den Mikrohohlräumen ausgehend von in Wasser löslichen Antibiotika-Salzen und in Wasser löslichen organischen Sulfaten oder Sulfonaten synthetisiert werden.

Der vorliegenden Erfindung liegt die Aufgabe zugrunde, ein unkompliziertes, kostengünstiges Verfahren zur antibiotischen Beschichtung von Körpern mit interkonnektierenden Mikrohohlraumsystemen zu entwickeln. Diese antibiotisch ausgerüsteten interkonnektierend-porösen Körper sollen als Implantate in der Human- und Veterinärmedizin zur Behandlung von Knochendefekten und gegebenenfalls zur Behandlung von Weichteildefekten Verwendung finden. Dabei wird eine kontinuierliche Antibiotika-Freisetzung aus der auf der inneren Oberfläche der interkonnektierenden Mikrohohlräume befindlichen antibiotischen Beschichtung über einen Zeitraum von mehreren Tagen bis zu mehreren Wochen angestrebt, damit eine mikrobielle Infektion im Bereich des zu behandelnden Knochendefekts und oder Weichteildefektes wirksam verhindert oder bekämpft werden kann.

Eine Aufgabe ist es, in einfacher Weise, unter Vermeidung von toxischen Lösungsmitteln und unter Verzicht auf polymere Bindemittel antibiotische Beschichtungen zu erzeugen, die eine Antibiotika-Freisetzung über einen Zeitraum von mehreren Tagen ermöglichen. Weiterhin wird angestrebt, ein für mehrere Antibiotika-Typen geeignetes Verfahren bereitzustellen. Es ist dabei von Vorteil, wenn die antibiotische Beschichtung auf der inneren Oberfläche von Körpern mit interkonnektierenden Mikrohohlräumen gut haftet und keine Gefahr besteht, dass die innterkonnektierenden Mikrohohlräume verstopft werden.

Die Aufgabe wird durch die Merkmale der unabhängigen Ansprüche gelöst. Vorteilhafte Ausgestaltungen ergeben sich aus den Unteransprüchen.

Der Erfindung liegt der überraschende Befund zugrunde, dass gut haftende, antibiotische Beschichtungen mit retardierender Wirkstofffreisetzung in den interkonnektierenden Mikrohohlräumen von Körpern gebildet werden, wenn zuerst eine wässrige Lösung 1, die mindestens eine in Wasser leicht lösliche antibiotische Komponente aus den Gruppen der Aminoglykosid-Antibiotika, der Tetracyclin-Antibiotika, der Lincosamid-Antibiotika, der 4-Chinolon-Antibiotika und der Chlorhexidine enthält, und anschließend nach Verdampfung und/oder Verdunstung des Wassers eine wässrige Lösung 2, die mindestens eine in Wasser lösliche amphiphile Komponente aus den Gruppen der Alkylsulfate, der Alkylsulfonate, der Alkylarylsulfate, Dialkylarylsulfate, Alkylarylsulfonate, Dialkylarylsulfonate, der Cycloalkylsulfate, der Cycloalkylsulfonate, der Alkylcycloalkylsulfate enthält, in geeigneter Weise, z.B. durch Tauchen, Sprühen oder Tropfen eingebracht werden.

Es werden auch antibiotische Beschichtungen gebildet, wenn in die Mikrohohlräume erst die wässrige Lösung 2 und anschließend nach Entfernung des Wassers die wässrige Lösung 1 durch Tauchen oder Sprühen oder Tropfen eingebracht wird.

Interkonnektierende Mikrohohlräume bedeuten in diesem Zusammenhang, dass Poren und auch unregelmäßig geformte Hohlräume durch Kanäle miteinander verbunden sind und nicht nach Art eines geschlossen-zelligen Schaumstoffs separat vorliegen. Bevorzugtes Material sind anorganische Materialien wie poröses Glas oder poröse Keramik.

Erfindungsgemäß ist, dass in den interkonnektierenden Mikrohohlraumsystemen von Festkörpem ein in Wasser gering lösliches Präzipitat einer oder mehrerer antibiotischer Substanzen aus den Gruppen der Aminoglykosid-Antibiotika, der Tetracyclin-Antibiotika, der Lincosamid-Antibiotika, 4-Chinolon-Antibiotika und der Chlorhexidine durch einen reziproken Salzaustausch zwischen mindestens einem in Wasser löslichen Salz aus der Gruppe Aminoglykosid-Antibiotika, der Tetracyclin-Antibiotika, der Lincosamid-Antibiotika, der 4-Chinolon-Antibiotika und der Chlorhexidine und mindestens einem in Wasser löslichen Salz aus der Gruppe der Alkylsulfate, der Alkylsulfonate, der Alkylarylsulfate, Dialkylarylsulfate, Alkylarylsulfonate, Dialkylarylsulfonate, der Cycloalkylsulfate, der Cycloalkylsulfonate, der Alkylcycloalkylsulfate synthetisiert wird und antibiotische Beschichtungen bilden. Die in den Mikrohohlräumen entstandenen antibiotischen Beschichtungen zeigen im wässrigen Milieu eine verzögerte Wirkstofffreisetzung über einen Zeitraum von mehreren Tagen bis Wochen. Insbesondere die Antibiotika-Präzipitate der Alkylsulfate und Alkylsulfonate fallen aus der wässrigen Lösung bei ihrer Synthese flockig als wachsartige, nichtkristalline Substanzen aus, die bei der Trocknung einen gewissen Verlauf zeigen und sich als Schicht auf Oberflächen auflagern. Sie haften überraschenderweise gut auf Glas-, Keramik- und Kunststoffoberflächen.

Im Sinne der Erfindung ist auch die Verwendung der Alkylsulfate, der Alkylsulfonate, der Alkylarylsulfate, Dialkylarylsulfate, Alkylarylsulfonate, Dialkylarylsulfonate, der Cycloalkylsulfate, der Cycloalkylsulfonate und der Alkylcycloalkylsulfate in der Säureform anstelle der Salzform.

Der besondere Vorteil des erfindungsgemäßen Verfahrens ist darin zu sehen, dass die in Wasser gering löslichen antibiotischen Präzipitate erst unter in situ Bedingungen in den interkonnektierenden Mikrohohlräumen entstehen und nicht vorher separat synthetisiert werden müssen. Mit dem Verfahren ist eine sehr kostengünstige, einfache antibiotische Beschichtung der inneren Oberfläche von porösen Körpern unterschiedlichster stofflicher Zusammensetzung realisierbar. Die in Wasser gering löslichen Präzipitate haften auf der Porenoberfläche und sind in den Mikrohohlräumen mechanisch geschützt. Dadurch kann auf zusätzliche polymere Bindemittel zur mechanischen Stabilisierung der Beschichtung verzichtet werden. Nach Auflösung der in Wasser gering löslichen Präzipitate verbleiben somit keine unerwünschten Hilfsstoffe in den Mikrohohlräumen. Das Verfahren ist insbesondere auch zur Erzeugung von antibiotischen Beschichtungen in mikroporösen Porensystemen geeignet.

Bevorzugt sind erfindungsgemäß in der wässrigen Lösung 1 als antibiotische Komponente Allomycin, Amicetin, Amikacin, Apramycin, Bekanamycin, Betamicin, Butirosin, Destomycin, Dibekacin, Dihydrostreptomycin, Flambamycin, Fortimycin A, Fortimycin B, Framycetin, Gentamicin, Hikizimycin, Homomycin, Hybrimycin, Hygromycin B, Kanamycin, Kasuhamycin, Lividomycin, Minosaminoycin, Neomycin, Netilmicin, Paromomycin, Parvulomycin, Puromycin A, Ribostamycin, Rimocidin, Ristosamin, Ristomycin, Sagamycin, Sisomicin, Sorbistin, Spectinomycin, Streptomycin, Tobramycin, Tunicamycin, Verdamycin aus der Gruppe der Aminoglykosid-Antibiotika.

Clindamycin und Lincomycin sind aus der Gruppe der Lincosamid-Antibiotika als antibiotische Komponente in der wässrigen Lösung 1 bevorzugt.

Tetracyclin, Chlortetracyclin, Oxytetracylin, Demethylchlortetracyclin, Methacyclin, Doxycyclin, Rolitetracyclin und Minocyclin sind aus der Gruppe der Tetracyclin-Antibiotika als antibiotische Komponente in der wässrigen Lösung 1 bevorzugt.

Ciprofloxacin, Moxifloxacin und Enfloxacin sind aus der Gruppe der 4-Chinolon-Antibiotika als antibiotische Komponente in der wässrigen Lösung 1 bevorzugt.

Aus der Gruppe der Chlorhexidine sind Chlorhexidin-di-chlorid, Chlorhexidin-di-acetat und Chlorhexidin-di-gluconat als antibiotische Komponente in der wässrigen Lösung 1 bevorzugt.

In der wässrigen Lösung 1 sind bevorzugt 0,1 bis 60 Masseprozent einer in Wasser leicht löslichen antibiotischen Komponente aus den Gruppen der Aminoglukosid-Antibiotika, der Lincosamid-Antibiotika, der Tetracyclin-Antibiotika, der 4-Chinolon-Antibiotika und der Chlorhexidine enthalten.

In der wässrigen Lösung 2 sind bevorzugt 0,1 bis 60 Masseprozent einer in Wasser löslichen amphiphilen Komponente aus den Gruppen der Alkylsulfate, der Alkylsulfonate, der Alkylarylsulfate, Dialkylarylsulfate, Alkylarylsulfonate, Dialkylarylsulfonate, der Cycloalkylsulfate, der Cycloalkylsulfonate, der Alkylcycloalkylsulfate enthalten.

Das Verhältnis der Stoffmenge der in Wasser leicht löslichen antibiotischen Komponente der wässrigen Lösung 1 zur Stoffmenge der in Wasser löslichen amphiphilen Komponente der wässrigen Lösung 2 beträgt zweckmäßig 1:1 bis 6:1.

In der wässrigen Lösung 1 liegt die antibiotische Komponente vorteilhaft in der protonierten Salzform vor, wobei Chlorid-Ionen, Bromid-Ionen, Hydrogensulfat-Ionen, Sulfat-Ionen, Dihydrogenphosphat-Ionen, Hydrogenphosphat-Ionen, Phosphat-Ionen, Acetat-Ionen, Succinat-Ionen und Lactat-lonen als Gegenionen bevorzugt sind.

Für das Einbringen der wässrigen Lösungen 1 und 2 in die Mikrohohlräume wird mit Vorteil die Kapillarwirkung genutzt, d. h. es kann beispielsweise durch vollständiges oder teilweises Eintauchen, durch Sprühen, Tropfen oder Träufeln erfolgen.

Zweckmäßig erfolgt im Fall des Gentamicins zunächst Einbringung einer wässrigen Lösung von beispielsweise Gentamicinsulfat in die Mikrohohlräume durch Tauchen oder Sprühen oder Tropfen, darauffolgend eine Trocknung zur Entfernung des Wassers aus den Poren, gefolgt von einer Einbringung einer wässrigen Lösung von Natriumdodecylsulfat und/oder einer wässrigen Lösung von Natriumdodecylsulfonat durch Tauchen oder Sprühen oder Tropfen.

Im Fall des Ciprofloxacins erfolgt zunächst eine Einbringung einer wässrigen Lösung von Ciprofloxacinhydrochlorid in die Poren durch Tauchen oder Sprühen oder Tropfen, eine darauffolgende Trocknung zur Entfernung des Wassers aus den Poren und eine nachfolgenden Einbringung einer wässrigen Lösung von Natriumdodecylbenzylsulfonat durch Tauchen oder Sprühen oder Tropfen.

Im Fall der Tetracycline geht man beispielsweise zweckmäßig so vor, dass eine Einbringung einer wässrigen Lösung von Tetracyclinhydrochlorid und/oder Chlortetracyclinhydrochlorid und/oder Minocyclinhydrochlorid und/oder Doxycyclinhydrochlorid in die Poren durch Tauchen oder Sprühen oder Tropfen, eine darauffolgende Trocknung zur Entfernung des Wassers aus den Poren und eine nachfolgenden Einbringung einer wässrigen Lösung von Natriumdodecylsulfat und/oder einer wässrigen Lösung von Natriumdodecylsulfonat durch Tauchen oder Sprühen erfolgt.

Man geht zweckmäßig so vor, dass nach dem Einbringen der ersten Lösung das Wasser im wesentlichen vollständig entfernt wird. Das kann beispielsweise durch Trocknung im Gasstrom oder durch Anwendung von Unterdruck oder auch thermisch geschehen. Auch Gefriertrocknung ist möglich und kann bei empfindlichen antibiotischen Wirkstoffen angezeigt sein. Durch die Art der Trocknung (Temperatur und Druckführung) kann auch die Beschaffenheit der antibiotischen Beschichtung beeinflusst werden. Die Art der Trocknung kann faserhaltigen Körpern mit interkonnektierenden Mikrohohlraumsystemen wie Vliesen, Filzen oder Gewirken angepasst werden.

Das Wasser kann nach Einbringung der ersten wässrigen in die Poren bei Normaldruck oder im Vakuum bei Temperaturen von -20°C bis 120°C teilweise oder vollständig entfernt werden.

Nach der Bildung der in Wasser gering löslichen Präzipitate können die Formkörper bei Normaldruck oder im Vakuum bei Temperaturen von -20°C bis 120°C getrocknet werden. Unter Vakuum wird hier der herkömmliche Unterdruck verstanden, der zur Entfernung des Wassers üblicherweise angewandt wird.

Als besonders vorteilhaft haben sich Natriumdodecylsulfat, Natriumdodecylsulfonat, Natriumtetradecylsulfat, Natriumtetradodecylsulfonat, Natriumhexadecylsulfat, Natriumhexadecylsulfonat, Natriumoctadecylsulfat, Natriumoctadecylsulfonat und Natriumdodecylbenzylsulfonat als in Wasser lösliche amphiphile Komponenten der wässrigen Lösung 2 erwiesen.

Die Körper mit interkonnektierenden Mikrohohlräumen können aus anorganischen oder organischen bzw. polymeren organischen Materialien aufgebaut sein, oder anorganisch-organische Composite-Materialien darstellen.

Im ersten Fall bestehen sie bevorzugt aus Hydroxylapatit, Tricalciumphosphat, Calciumcarbonat, Calciumsulfat, resorbierbarem Glas, resorbierbarer Glaskeramik oder aus Mischungen dieser Materialien.

Im zweiten Fall sind sie z. B. aus Polymeren aufgebaut, etwa auf Basis von L-Milchsäure und/oder D-Milchsäure und/oder Glykolsäure und/oder 2-Hydroxyethyl-oxyessigsäure. Solche Polymer-Systeme sind beispielsweise unter dem Handelsnamen Resomer® bei der Fa. Boehringer Ingelheim erhältlich.

Die Körper mit interkonnektierenden Mikrohohlräumen können auch aus Metall oder Metall-Legierungen, insbesondere Titan, Titanlegierungen oder Edelstahl gebildet sein. Unter metallischen Körpern mit interkonnektierenden Mikrohohlräumen werden solche metallischen Körper verstanden, die an ihrer Oberfläche Mikrohohlräume enthalten, die miteinander verbunden sind, und es werden ebenfalls metallische Körper dazugerechnet, deren Oberfläche durchs Sandstrahlung so aufgerauht wurde, dass diese offene, miteinander verbundene Hohlräume in der Metalloberfläche aufweisen.

Erfindungsgemäß ist es ferner möglich, dass die Körper mit interkonnektierenden Mikrohohlraumsystemen die Form von Vliesen, Filzen, Geweben und Gewirken haben.

Außerdem ist es erfindungsgemäß möglich, dass antibiotische Beschichtungen die Volumina der interkonnektierenden Mikrohohlräume von Festkörpern nicht vollständig ausfüllen.

Es kann vorteilhaft sein, zur Erhöhung der Löslichkeit organische, mit Wasser mischbare Lösungsmittel wie Methanol, Ethanol, Isopropanol, N,N-Dimethylformamid und/oder Dimethylsulfoxid als Kosolvens zu der wässrigen Lösung 1 oder 2 zuzusetzen.

Im Sinne der Erfindung ist, dass die antibiotisch-beschichteten, interkonnektierend-porösen, Körper als Implantate verwendet werden.

Auch im Sinne der Erfindung ist, dass die Lösung 2 in die Körper mit interkonnekierendenen Mikrohohlraumsystemen eingebracht wird und nach der Entfernung des Wassers, die so behandelten Körper mit interkonnektierenden Mikrohohlraumsystemen als Implantatmaterial verwendet werden, in die erst unmittelbar vor der Implantation die Lösung 1 eingebracht wird. Dadurch ist es möglich, nach einem zuvor durchgeführten Antibiogramm, eine antibiotische Beschichtung mit einem für die jeweiligen Mikroorganismen am geeignetsten Antibiotika vorzunehmen.

Ebenfalls im Sinne der Erfindung ist, dass die Lösung 2, die eine amphiphile Komponente aus den Gruppen der Trialkylammoniumsalze, der Dialkylammoniumsalze, der Dialkylaarylammoniumsalze, der Alkylarylammoniumsalze, der Diarylammoniumsalze und der Triarylammoniumsalze enthält, in die Körper mit interkonnektierenden Mikrohohlraumsystemen eingebracht wird und nach Entfernung des Wassers die so behandelten Körper mit interkonnektierenden Mikrohohlraumsystemen als Implantatmaterial verwendet werden, in die erst unmittelbar vor der Implantation eine wässrige Lösung eines sauren Antibiotikums, das Carboxyl- und oder SulfatGruppen enthält, eingebracht wird.

Die nachstehenden Beispiele 1 bis 8 dienen der Erläuterung der Erfindung, ohne sie zu beschränken.

### Beispiele

Als Körper mit interkonnektierenden Porensystemen wurden quaderförmige, resorbierbare Phosphatgläser mit den Abmessungen von 20x20x10 mm für die Beispiele 1 bis 8 verwendet. Sie hatten eine Gesamt-Porosität von 65 Volumenprozent.

### Allgemeine Vorgehensweise bei der Präparation der Beispiele 1 bis 5: :

Es wurden 50 bis 100 mg Gentamicinsulfat in 1 g bidest. Wasser gelöst (Lösung 1). Separat wurden 50 bis 150 mg Natriumdodecylsulfat in 1 g Wasser in Lösung gebracht (Lösung 2). In die Poren der quaderförmigen Phosphatgläser wurden zuerst die zuvor hergestellten wässrigen Gentamicinsulfat-Lösungen getropft. Die Probekörper saugten die Gentamicinsulfat-Lösungen auf. Danach wurde das in den Poren befindliche Wasser durch Trocknen über wasserfreiem Calciumchlorid entfernt. Dann wurden die vorbereiteten wässrigen Natriumdodecylsulfat-Lösungen in die Poren der getrockneten Phosphatgläser getropft. Die Trocknung der Probekörper erfolgte ebenfalls über wasserfreiem Calciumchlorid bis zur Massekonstanz.

**Tab. 1: Zusammensetzungen der Lösung 1 und der Lösung 2 sowie Auswaagen der unbeschichteten und beschichteten Probekörper der Beispiele 1 bis 5.**

| Beispiel Nr. | Zusammensetzung der Lösung 1 | Zusammensetzung der Lösung 2 | Masse der Probekörper vor Beschichtung [mg] | Masse der Probekörper nach Beschichtung [mg] | Masse der Beschichtung [mg] |
|---|---|---|---|---|---|
| 1 | 50 mg GS | 50mg SDS | 3643 | 3734 | 91 |
| | 1000 mg H₂O | 1000 mg H₂O | | | |
| 2 | 50 mg GS | 100mg SDS | 4186 | 4323 | 137 |
| | 1000 mg H₂O | 1000 mg H₂O | | | |
| | 50 mg GS | 150mg SDS | 3244 | 3430 | 186 |
| | 1000 mg H₂O | 1000 mg H₂O | | | |
| 4 | 100 mg GS | 100mg SDS | 3384 | 3581 | 197 |
| | 1000 mg H₂O | 1000mg H₂O | | | |
| 5 | 100 mg GS | 200mg SDS | 3335 | 3615 | 280 |
| | 1000 mg H₂O | 1000 mg H₂O | | | |

| | | | | | |
|---|---|---|---|---|---|
| GS: Gentamicinsulfat (AK=628) SDS: Natriumdodecylsulfat | | | | | |

### Antibiotika-Freisetzung der Probekörper der Beispiele 1 bis 5:

Die in den Beispielen 1 bis 5 hergestellten Formkörper wurden in jeweils 20 ml physiologische Kochsalzlösung eingebracht und in dieser bei 37 °C über einen Zeitraum von 28 Tagen gelagert. Die Probennahme erfolgte nach 1, 2, 3, 6, 9, 12, 15, 21 und 28 Tagen Lagerungszeit. Nach jeder Probennahme wurde das Freisetzungsmedium vollständig durch frisches Medium, ersetzt. Die Antibiotika-Wertbestimmung wurde mit einem Agardiffusionstest unter Verwendung von Bacillus subtilis ATCC 6633 als Testkeim durchgeführt. Die Ergebnisse sind in Tab. 2 dargestellt und zeigen deutlich, dass die beschichteten Probekörper kontinuierlich über einen Zeitraum von 28 Tagen Gentamicin freisetzen. Nach 28 Tagen wurde die Untersuchung abgebrochen. Die beschichteten Formkörper stellen somit Gentamicin-Depotformen dar, die entsprechend der Aufgabe der Erfindung, über einen Zeitraum von vier Wochen, Gentamicin in das umgebende wässrige Milieu abgeben.

**Tab. 2: Ergebnisse der mikrobiellen Bestimmung der Gentamicin-Freisetzung der beschichteten Probekörper der Beispiele 1 bis 5 in Abhängigkeit von der Lagerungszeit der Probekörper in physiologischer Kochsalzlösung bei 37 °C.**

| Beispiel Nr. | Gentamicin-Freisetzung (kumuliert, als Gentamicinsulfat AK=628) | | | | | | | | |
|---|---|---|---|---|---|---|---|---|---|
| | [mg] | | | | | | | | |
| | Freisetzungszeit [d] | | | | | | | | |
| | 1 | 2 | 3 | 6 | 9 | 12 | 15 | 21 | 28 |
| 1 | 8,8 | 10,5 | 12,1 | 13,8 | 15,2 | 16,4 | 17,5 | 18,6 | 19,4 |
| 2 | 1,2 | 1,5 | 1,8 | 2,1 | 2,4 | 3,0 | 3,8 | 4,7 | 5,5 |
| 3 | 1,2 | 1,4 | 1,6 | 1,8 | 2,0 | 2,2 | 2,5 | 2,7 | 3,1 |
| 4 | 27,5 | 30,4 | 32,8 | 35,3 | 36,8 | 38,2 | 39,2 | 40,4 | 41,4 |
| 5 | 3,0 | 3,2 | 3,6 | 3,8 | 4,0 | 4,2 | 5,0 | 5,3 | 5,7 |

### Allgemeine Vorgehensweise bei der Präparation der Beispiele 6 bis 8:

Die Vorgehensweise ist analog der bei den Beispielen 1 bis 5 angewendeten Präparation. Nur wurden hierbei jeweils die Lösungen 2 auf 80 bis 90 °C vor dem Auftropfen auf die Probekörper erwärmt. Die Untersuchung der Gentamicin-Freisetzung wurde in gleicher Weise wie bei den Beispielen 1 bis 5 vorgenommen.

**Tab. 3: Zusammensetzungen der Lösung 1 und der Lösung 2 sowie Auswaagen der unbeschichteten und beschichteten Probekörper der Beispiele 6 bis 8.**

| Beispiel Nr. | Zusammensetzung der Lösung 1 | Zusammensetzung der Lösung 2 | Masse der Probekörper vor Beschichtung | Masse der Probekörper nach Beschichtung | Masse der Beschichtung |
|---|---|---|---|---|---|
| | | | [mg] | [mg] | [mg] |
| 6 | 50 mg GS | 50 mg NDS | 3945 | 4041 | 96 |
| | 1000 mg H₂O | 1000 mg H₂O | | | |
| 7 | 100 mg GS | 100 mg NDS | 4249 | 4447 | 198 |
| | 1000 mg H₂O | 1000 mg H₂O | | | |
| 8 | 50 mg GS | 150 mg NDS | 3378 | 3575 | 197 |
| | 1000 mg H₂O | 1000 mg H₂O | | | |

| | | | | | |
|---|---|---|---|---|---|
| GS: Gentamicinsulfat (AK=628) NDS: Natriumdodecylsulfonat | | | | | |

### Antibiotika-Freisetzung der Probekörper der Beispiele 6 bis 8:

Die Antibiotika-Freisetzung wurde in gleicher Weise wie bei den Beispielen 1 bis 5 durchgeführt und die Gentamicin-Wertbestimmung erfolgte in analoger Weise mikrobiell mit dem bacillus subtilis ATCC 6633 als Testkeim. Die Ergebnisse der Freisetzungsuntersuchung sind in Tab. 4 dargestellt. Aus diesen Ergebnissen ist ersichtlich, dass die mit wässriger Gentamicinsulfat-Lösung und mit wässriger Natriumdodecylsulfonat-Lösung beschichteten Probekörper ebenfalls eine verzögerte Gentamicin-Freisetzung über einen Zeitraum von 28 Tagen zeigten. Nach 28 Tagen wurden die Freisetzungsversuche abgebrochen. Ein Vergleich der Masse des zur antibiotischen Beschichtung eingesetzten Gentamicinsulfats mit der Masse des freigesetzten Gentamicins zeigt, dass sich ein nicht unbeträchlicher Anteil des Gentamicins nach 28 Tagen noch in der Beschichtung befindet. Das Beispiel Nr. 8 zeigt deutlich, dass durch einen erhöhten Anteil an Dodecylsulfonat in der Beschichtung, die Gentamicin Freisetzung innerhalb des ersten Tages deutlich gesenkt werden kann.

**Tab. 4: Ergebnisse der mikrobiellen Bestimmung der Gentamicin-Freisetzung der Probekörper der Beispiele 6 bis 8 in Abhängigkeit von der Lagerungszeit der Probekörper in physiologischer Kochsalzlösung bei 37 °C.**

| Beispiel Nr. | Gentamicin-Freisetzung (kumuliert, als Gentamicinsulfat AK=628) | | | | | | | | |
|---|---|---|---|---|---|---|---|---|---|
| | [mg] | | | | | | | | |
| | Freisetzungszeit [d] | | | | | | | | |
| | 1 | 2 | 3 | 6 | 9 | 12 | 15 | 21 | 28 |
| 6 | 16,9 | 20,8 | 23,4 | 24,9 | 26,4 | 27,6 | 28,2 | 29,5 | 31,0 |
| 7 | 19,1 | 24,4 | 29,5 | 33,7 | 35,8 | 37,9 | 39,9 | 42,1 | 43,5 |
| 8 | 2,7 | 4,4 | 5,2 | 5,7 | 6,2 | 6,6 | 7,0 | 7,6 | 8,2 |

## Patentansprüche

1. Verfahren zur antibiotischen Beschichtung von Körpern mit interkonnektierenden Mikrohohlräumen, **dadurch gekennzeichnet, dass** in die Mikrohohlräume eine wässrige Lösung 1, die mindestens eine in Wasser leicht lösliche antibiotische Komponente aus den Gruppen der Aminoglykosid-Antibiotika, der Tetracyclin-Antibiotika, der Lincosamid-Antibiotika, der 4-Chinolon-Antibiotika und der Chlorhexidine enthält, und eine wässrige Lösung 2, die mindestens eine in Wasser lösliche amphiphile Komponente aus den Gruppen der Alkylsulfate, der Alkylsulfonate, der Alkylarylsulfate, Dialkylarylsulfate, Alkylarylsulfonate, Dialkylarylsulfonate, der Cycloalkylsulfate, der Cycloalkylsulfonate, der Alkylcycloalkylsulfate enthält, eingebracht werden, wobei zwischen dem Einbringen der Lösungen 1 und 2 das Wasser im wesentlichen entfernt wird, und wobei aus den Komponenten der Lösungen 1 und 2 in den Mikrohohlräumen eine Beschichtung gebildet wird, die aus einem in Wasser gering löslichen Präzipitat besteht.

2. Verfahren nach Anspruch 1, **dadurch gekennzeichnet, dass** in die Mikrohohlräume erst die wässrige Lösung 1 und anschließend nach Entfernung des Wassers die wässrige Lösung 2 eingebracht wird.

3. Verfahren nach Anspruch 1, **dadurch gekennzeichnet, dass** in die Mikrohohlräume erst die wässrige Lösung 2 und anschließend nach Entfernung des Wassers die wässrige Lösung 1 eingebracht wird.

4. Verfahren nach Anspruch 1, **dadurch gekennzeichnet, dass** in Lösung 1 mindestens eine antibiotische Substanz aus der Gruppe: Allomycin, Amicetin, Amikacin, Apramycin, Bekanamycin, Betamicin, Butirosin, Destomycin, Dibekacin, Dihydrostreptomycin, Flambamycin, Fortimycin A, Fortimycin B, Framycetin, Gentamicin, Hikizimycin, Homomycin, Hybrimycin, Hygromycin B, Kanamycin, Kasuhamycin, Lividomycin, Minosaminoycin, Neomycin, Netilmicin, Paromomycin, Parvulomycin, Puromycin A, Ribostamycin, Rimocidin, Ristosamin, Ristomycin, Sagamycin, Sisomicin, Sorbistin, Spectinomycin, Streptomycin, Tobramycin, Tunicamycin, Verdamycin, Clindamycin und Lincomycin , Tetracyclin, Chlortetracyclin, Oxytetracylin, Dimethylchlortetracyclin, Methacyclin, Doxycyclin, Rolitetracyclin, Minocyclin, Ciprofloxacin, Enfloxacin, Chlorhexidin-di-hydrochlorid, Chlorhexidin-diacetat und Chlorhexidin-di-gluconat eingesetzt wird.

5. Verfahren nach Anspruch 1, **dadurch gekennzeichnet, dass** die in Wasser leicht lösliche antibiotische Komponente zu 0,1 bis 60 Masseprozent in der wässrigen Lösung 1 enthalten ist.

6. Verfahren nach einem der Ansprüche 1 bis 5, **dadurch gekennzeichnet, dass** die in Wasser lösliche amphiphile Komponente in der wässrigen Lösung 2 zu 0,1 bis 60 Masseprozent enthalten ist.

7. Verfahren nach einem der Ansprüche 1 bis 6, **dadurch gekennzeichnet, dass** das Verhältnis der Stoffmenge der in Wasser leicht löslichen antibiotischen Komponente der wässrigen Lösung 1 zur Stoffmenge der in Wasser löslichen amphiphilen Komponente der wässrigen Lösung 2 von 1:1 bis 6:1 beträgt.

8. Verfahren nach einem der Ansprüche 1 bis 7, **dadurch gekennzeichnet, dass** die antibiotische Komponente in der wässrigen Lösung 1 in der protonierten Salzform vorliegt.

9. Verfahren nach Anspruch 8, **dadurch gekennzeichnet, dass** Chlorid-lonen, Bromid-lonen, Hydrogensulfat-lonen, Sulfat-lonen, Dihydrogenphosphat-lonen, Hydrogenphosphat-lonen, Phosphat-lonen, Acetat-lonen, Succinat-lonen und Lactat-lonen als Gegenionen eingesetzt werden.

10. Verfahren nach einem der Ansprüche 1 bis 9, **dadurch gekennzeichnet, dass** die Lösungen 1 und/oder 2 durch Tauchen, Sprühen oder Tropfen in die Mikrohohlräume eingebracht werden.

11. Verfahren nach einem der Ansprüche 1 bis 10, **dadurch gekennzeichnet, dass** eine Einbringung einer wässrigen Lösung von Gentamicinsulfat in die Porensysteme durch Tauchen oder Sprühen oder Tropfen, eine darauffolgende Trocknung zur Entfernung des Wassers aus den Mikrohohlräumen und eine nachfolgenden Einbringung einer wässrigen Lösung von Natriumdodecylsulfat und/oder einer wässrigen Lösung von Natriumdodecylsulfonat durch Tauchen oder Sprühen oder Tropfen erfolgt, oder dass eine Einbringung einer wässrigen Lösung von Ciprofloxacinhydrochlorid in die Mikrohohlräume durch Tauchen oder Sprühen oder Tropfen, eine darauffolgenden Trocknung zur Entfernung des Wassers aus den Mikrohohlräumen und eine nachfolgende Einbringung einer wässrigen Lösung von Natriumdodecylbenzylsulfonat durch Tauchen oder Sprühen oder Tropfen erfolgt, oder dass eine Einbringung einer wässrigen Lösung von Tetracyclinhydrochlorid und oder Chlortetracyclinhydrochlorid und oder Minocyclinhydrochlorid und oder Doxycyclinhydrochlorid in die Mikrohohlräume durch Tauchen oder Sprühen oder Tropfen, eine darauffolgenden Trocknung zur Entfernung des Wassers aus den Mikrohohlräumen und eine nachfolgenden Einbringung einer wässrigen Lösung von Natriumdodecylsulfat und/oder einer wässrigen Lösung von Natriumdodecylsulfonat durch Tauchen oder Sprühen erfolgt.

12. Verfahren nach mindestens einem der Ansprüche 1 bis 11, **dadurch gekennzeichnet, dass** nach Einbringung der ersten wässrigen Lösung in die Mikrohohlräume das Wasser durch Trocknung bei Normaldruck oder im Vakuum bei Temperaturen von -20°C bis 120°C teilweise oder vollständig entfernt wird.

13. Verfahren nach einem der Ansprüche 1 bis 12, **dadurch gekennzeichnet, dass** nach der Bildung des in Wasser gering löslichen Präzipitats die Formkörper bei Normaldruck oder im Vakuum bei Temperaturen von -20°C bis 120°C getrocknet werden.

14. Verfahren nach einem der Ansprüche 1 bis 13, **dadurch gekennzeichnet, dass** mindestens ein Stoff aus der Gruppe: Natriumdodecylsulfat, Natriumdodecylsulfonat, Natriumtetradecylsulfat, Natriumtetradodecylsulfonat, Natriumhexadecylsulfat, Natriumhexadecylsulfaonat, Natriumoctadecylsulfat, Natriumoctadecylsulfonat und Natriumdodecylbenzylsulfonat als in Wasser lösliche amphiphile Komponenten der wässrigen Lösung 2 eingesetzt wird.

15. Verfahren nach einem der Ansprüche 1 bis 14, **dadurch gekennzeichnet, dass** die Mikrohohlräume Poren sind.

16. Verfahren nach einem der Ansprüche 1 bis 15, **dadurch gekennzeichnet, dass** die Körper mit interkonnektierenden Mikrohohlraumsystemen aus Hydroxylapatit, Tricalciumphosphat, Calciumcarbonat, Calciumsulfat, resorbierbarem Glas und resorbierbarer Glaskeramik bestehen.

17. Verfahren nach einem der Ansprüche 1 bis 14, **dadurch gekennzeichnet, dass** die Körper mit interkonnektierenden Mikrohohlräumen aus Polymeren bestehen, die auf L-Milchsäure und oder D-Milchsäure und oder Glykolsäure und oder 2-Hydroxyethyl-oxyessigsäure basieren.

18. Verfahren nach Anspruch 17, **dadurch gekennzeichnet, dass** die interkonnektierend-porösen Körper in Form von Schwämmen, Schaumstoffkörpern, Vliesen, Filzen, Geweben oder Gewirken vorliegen.

19. Verfahren nach einem der Ansprüche 1 bis 18, **dadurch gekennzeichnet, dass** die Körper aus Titan, Titanlegierungen oder Edelstahl bestehen.

20. Verfahren nach einem der Ansprüche 1 bis 19, **dadurch gekennzeichnet, dass** die erfindungsgemäße antibiotische Beschichtung die Volumina der interkonnektierenden Mikrohohlräume nicht vollständig ausfüllt.

21. Verfahren nach einem der Ansprüche 1 bis 20, **dadurch gekennzeichnet, dass** Methanol, Ethanol, Isopropanol, N,N-Dimethylformamid und/oder Dimethylsulfoxid als Kosolvens zu der wässrigen Lösung 1 und/oder zu der wässrigen Lösung 2 zugesetzt werden.

22. Verfahren nach einem der Ansprüche 1 bis 21, **dadurch gekennzeichnet, dass** die antibiotisch-beschichteten, Körper mit interkonnektierenden Mikrohohlräumen, als Implantate verwendet werden.

23. Verfahren nach einem der Ansprüche 1 bis 22, **dadurch gekennzeichnet, dass** die Lösung 2 in die Körper mit interkonnekierendenen Mikrohohlraumsystemen eingebracht wird und nach der Entfernung des Wassers die so behandelten Körper mit interkonnektierenden Mikrohohlraumsystemen als Implantatmaterial verwendet werden, in die erst unmittelbar vor der Implantation die Lösung 1 eingebracht wird.

## Claims

1. Process for the antibiotic coating of bodies having interconnecting micro-cavities, **characterized in that** an aqueous solution 1 which contains at least one antibiotic component freely soluble in water from the groups consisting of the aminoglycoside antibiotics, the tetracycline antibiotics, the lincosamide antibiotics, the 4-quinolone antibiotics and the chlorhexidines, and an aqueous solution 2 which contains at least one water-soluble amphiphilic component from the groups consisting of the alkylsulphates, the alkanesulphonates, the alkylarylsulphates, dialkylarylsulphates, alkylarylsulphonates, dialkylarylsulphonates, the cycloalkyl sulphates, the cycloalkanesulphonates and the alkylcycloalkylsulphates are introduced into the microcavities, the water being substantially removed between the introduction of solutions 1 and 2, and a coating which consists of a precipitate which is sparingly soluble in water being formed from the components of solutions 1 and 2 in the microcavities.

2. Process according to Claim 1, **characterized in that** first the aqueous solution 1 and then, after removal of the water, the aqueous solution 2 is introduced into the microcavities.

3. Process according to Claim 1, **characterized in that** first the aqueous solution 2 and then, after removal of the water, the aqueous solution 1 is introduced into the microcavities.

4. Process according to Claim 1, **characterized in that** at least one antibiotic substance from the group: allomycin, amicetin, amikacin, apramycin, bekanamycin, betamicin, butirosin, destomycin, dibekacin, dihydrostreptomycin, flambamycin, fortimycin A, fortimycin B, framycetin, gentamicin, hikizimycin, homomycin, hybrimycin, hygromycin B, kanamycin, kasuhamycin, lividomycin, minosaminomycin, neomycin, netilmicin, paromomycin, parvulomycin, puromycin A, ribostamycin, rimocidin, ristosamine, ristomycin, sagamycin, sisomicin, sorbistin, spectinomycin, streptomycin, tobramycin, tunicamycin, verdamycin, clindamycin and lincomycin, tetracycline, chlorotetracycline, oxytetracycline, dimethylchlorotetracycline, methacycline, doxycycline, rolitetracycline, minocycline, ciprofloxacin, enfloxacin, chlorhexidine dihydrochloride, chlorhexidine diacetate and chlorhexidine digluconate is used in solution 1.

5. Process according to Claim 1, **characterized in that** the antibiotic component which is freely soluble in water is present in an amount of 0.1 to 60% percent by mass in the aqueous solution 1.

6. Process according to any of Claims 1 to 5, **characterized in that** the water-soluble amphiphilic component is present in the aqueous solution 2 in an amount of 0.1 to 60 percent by mass.

7. Process according to any of Claims 1 to 6, **characterized in that** the ratio of the amount of that antibiotic component of the aqueous solution 1 which is freely soluble in water to the amount of the water-soluble amphiphilic component of the aqueous solution 2 is from 1:1 to 6:1.

8. Process according to any of Claims 1 to 7, **characterized in that** the antibiotic component is present in protonated salt form in the aqueous solution 1.

9. Process according to Claim 8, **characterized in that** chloride ions, bromide ions, hydrogen sulphate ions, sulphate ions, dihydrogen phosphate ions, hydrogen phosphate ions, phosphate ions, acetate ions, succinate ions and lactate ions are used as counter-ions.

10. Process according to any of Claims 1 to 9, **characterized in that** the solutions 1 and/or 2 are introduced into the microcavities by immersion, spraying or dripping.

11. Process according to any of Claims 1 to 10, **characterized in that** an aqueous solution of gentamicin sulphate is introduced into the pore system by immersion or spraying or dripping, subsequent drying for removal of the water from the microcavities is effected and an aqueous solution of sodium dodecyl sulphate and/or an aqueous solution of sodium dodecanesulphonate is then introduced by immersion or spraying or dripping, or **in that** an aqueous solution of ciprofloxacin hydrochloride is introduced into the microcavities by immersion or spraying or dripping, subsequent drying for removal of the water from the microcavities is effected and an aqueous solution of sodium dodecylbenzenesulphonate is then introduced by immersion or spraying or dripping, or **in that** an aqueous solution of tetracycline hydrochloride and/or chlorotetracycline hydrochloride and/or monocycline hydrochloride and/or doxycycline hydrochloride is introduced into the microcavities by immersion or spraying or dripping, subsequent drying for removal of the water from the microcavities is effected and an aqueous solution of sodium dodecylsulphate and/or an aqueous solution of sodium dodecanesulphonate is then introduced by immersion or spraying.

12. Process according to at least one of Claims 1 to 11, **characterized in that**, after introduction of the first aqueous solution into the microcavities, the water is partly or completely removed by drying at atmospheric pressure or in vacuo at temperatures of -20°C to 120°C.

13. Process according to any of Claims 1 to 12, **characterized in that**, after the formation of the precipitate which is sparingly soluble in water, the shaped bodies are dried at atmospheric pressure or in vacuo at temperatures of -20°C to 120°C.

14. Process according to any of Claims 1 to 13, **characterized in that** at least one substance from the group: sodium dodecylsulphate, sodium dodecanesulphonate, sodium tetradecylsulphate, sodium tetradecanesulphonate, sodium hexadecylsulphate, sodium hexadecanesulphonate, sodium octadecylsulphate, sodium octadecanesulphonate and sodium dodecylbenzenesulphonate is used as water-soluble amphiphilic components of the aqueous solution 2.

15. Process according to any of Claims 1 to 14, **characterized in that** the microcavities are pores.

16. Process according to any of Claims 1 to 15, **characterized in that** the bodies having interconnecting microcavity systems consist of hydroxylapatite, tricalcium phosphate, calcium carbonate, calcium sulphate, resorbable glass and resorbable glass ceramic.

17. Process according to any of Claims 1 to 14, **characterized in that** the bodies having interconnecting microcavities consist of polymers which are based on L-lactic acid and/or D-lactic acid and/or glycolic acid and/or 2-hydroxyethyloxyacetic acid.

18. Process according to Claim 17, **characterized in that** the bodies having interconnecting pores are present in the form of sponges, foam bodies, nonwovens, felts, woven fabrics or knitted fabrics.

19. Process according to any of Claims 1 to 18, **characterized in that** the bodies consist of titanium, titanium alloys or stainless steel.

20. Process according to any of Claims 1 to 19, **characterized in that** the antibiotic coating according to the invention does not completely fill the volumes of the interconnecting microcavities.

21. Process according to any of Claims 1 to 20, **characterized in that** methanol, ethanol, isopropanol, N,N-dimethylformamide and/or dimethyl sulphoxide are added as co-solvents to the aqueous solution 1 and/or to the aqueous solution 2.

22. Process according any of Claims 1 to 21, **characterized in that** the antibiotically coated bodies having interconnecting microcavities are used as implants.

23. Process according to any of Claims 1 to 22, **characterized in that** the solution 2 is introduced into the bodies having interconnecting microcavity systems and, after removal of the water, the bodies thus treated and having interconnecting microcavity systems are used as implant material into which the solution 1 is introduced only directly before implantation.

## Revendications

1. Procédé pour un revêtement antibiotique de corps présentant des micro-espaces creux interconnectés, **caractérisé en ce que** l'on introduit dans les micro-espaces creux une solution aqueuse 1 qui contient au moins un constituant antibiotique facilement soluble dans l'eau choisi parmi les antibiotiques d'aminoglycoside, les antibiotiques de tétracycline, les antibiotiques de lincosamide, les antibiotiques de 4-quinolone et la chlorhexidine, et une solution aqueuse 2 qui contient au moins un constituant amphiphile soluble dans l'eau choisi parmi les sulfates d'alkyle, les sulfonates d'alkyle, les sulfates d'alkylaryle, les sulfates de dialkylaryle, les sulfonates d'alkylaryle, les sulfonates de dialkylaryle, les sulfates de cycloalkyle, les sulfonates de cycloalkyle, les sulfates d'alkylcycloalkyle, l'eau étant en général éliminée entre l'introduction des solutions 1 et 2, et un revêtement étant formé à partir des constituants des solutions 1 et 2 dans les micro-espaces creux, lequel revêtement est constitué d'un précipité peu soluble dans l'eau.

2. Procédé selon la revendication 1, **caractérisé en ce que** l'on introduit dans les micro-espaces creux tout d'abord la solution aqueuse 1 et ensuite, après l'élimination de l'eau, la solution aqueuse 2.

3. Procédé selon la revendication 1, **caractérisé en ce que** l'on introduit dans les micro-espaces creux tout d'abord la solution aqueuse 2 et, ensuite après l'élimination de l'eau, la solution aqueuse 1.

4. Procédé selon la revendication 1, **caractérisé en ce que** l'on utilise dans la solution 1 au moins une substance antibiotique choisie parmi : l'allomycine, l'amicétine, l'amikacine, l'apramycine, la békanamycine, la bétamycine, la butirosine, la destomycine, la dibékacine, la dihydrostreptomycine, la flambamycine, la fortimycine A, la fortimycine B, la framycétine, la gentamicine, l'hikizimycine, l'homomycine, l'hybrimycine, l'hygromycine B, la kanamycine, la kasuhamycine, la lividomycine, la minosaminonoycine, la néomycine, la nétilmycine, la paromomycine, la parvulomycine, la puromycine A, la ribostamycine, la rimocidine, la ristosamine, la ristomycine, la sagamycine, la sisomicine, la sorbistine, la spectinomycine, la streptomycine, la tobramycine, la tunicamycine, la verdamycine, la clindamycine et la lincomycine, la tétracycline, la chlorotétracycline, l'oxytétracycline, la diméthylchlorotétracycline, la méthacycline, la doxycycline, la rolitétracycline, la minocycline, la ciprofloxacine, l'enfloxacine, le dihydrochlorure de chlorhexidine, le diacétate de chlorhexidrine et le di-gluconate de chlorhexidine.

5. Procédé selon la revendication 1, **caractérisé en ce que** le constituant antibiotique facilement soluble dans l'eau est contenu jusqu'à de 0,1 à 60 % en masse dans la solution aqueuse 1.

6. Procédé selon l'une quelconque des revendications 1 à 5, **caractérisé en ce que** le constituant amphiphile soluble dans l'eau est contenu dans la solution aqueuse 2 jusqu'à de 0,1 à 60 % en masse.

7. Procédé selon l'une quelconque des revendications 1 à 6, **caractérisé en ce que** le rapport de la quantité de matière du constituant antibiotique facilement soluble dans l'eau de la solution aqueuse 1 à la quantité de matière du constituant amphiphile soluble dans l'eau de la solution aqueuse 2 est compris entre 1:1 et 6:1.

8. Procédé selon l'une quelconque des revendications 1 à 7, **caractérisé en ce que** le constituant antibiotique est présent dans la solution aqueuse 1 dans la forme de sel protoné.

9. Procédé selon la revendication 8, **caractérisé en ce que** l'on utilise des ions chlorure, des ions bromure, des ions hydrogénosulfonate, des ions sulfate, des ions dihydrogénophosphate, des ions hydrogénophosphate, des ions phosphate, des ions acétate, des ions succinate et des ions lactate comme contre-ions.

10. Procédé selon l'une quelconque des revendications 1 à 9, **caractérisé en ce que** l'on introduit les solutions 1 et/ou 2 par immersion, pulvérisation ou addition goutte à goutte dans les micro-espaces creux.

11. Procédé selon l'une quelconque des revendications 1 à 10, **caractérisé en ce que** l'on réalise une introduction d'une solution aqueuse de sulfate de gentamicine dans les systèmes de pores par immersion ou pulvérisation ou addition goutte à goutte, un séchage subséquent pour éliminer l'eau des micro-espaces creux et une introduction subséquente d'une solution aqueuse de dodécylsulfate de sodium et/ou d'une solution aqueuse de dodécylsulfonate de sodium par immersion ou pulvérisation ou addition goutte à goutte, ou **en ce que** l'on réalise une introduction d'une solution aqueuse d'hydrochlorure de ciprofloxacine dans les micro-espaces creux par immersion ou pulvérisation ou addition goutte à goutte, un séchage subséquent pour éliminer l'eau des micro-espaces creux et une introduction subséquente d'une solution aqueuse de dodécylbenzylsulfonate de sodium par immersion ou pulvérisation ou addition goutte à goutte, ou **en ce que** l'on réalise une introduction d'une solution aqueuse d'hydrochlorure de tétracycline ou d'hydrochlorure de chlorotétracycline et/ou d'hydrochlorure de minocycline et/ou d'hydrochlorure de doxycycline dans les micro-espaces creux par immersion ou pulvérisation ou addition goutte à goutte, un séchage subséquent pour éliminer l'eau des micro-espaces creux et une introduction subséquente d'une solution aqueuse de dodécylsulfate de sodium et/ou d'une solution aqueuse de dodécylsulfonate de sodium par immersion ou pulvérisation.

12. Procédé selon au moins l'une quelconque des revendications 1 à 11, **caractérisé en ce que** l'on élimine partiellement ou totalement, après l'introduction de la première solution aqueuse dans les micro-espaces creux, l'eau par séchage à pression atmosphérique ou sous vide à des températures de -20°C à 120°C.

13. Procédé selon l'une quelconque des revendications 1 à 12, **caractérisé en ce qu'**après la formation du précipité faiblement soluble dans l'eau on sèche les corps moulés à pression atmosphérique ou sous vide à des températures de -20°C à 120°C.

14. Procédé selon l'une quelconque des revendications 1 à 13, **caractérisé en ce que** l'on utilise au moins une matière choisie parmi : le dodécylsulfate de sodium, le dodécylsulfonate de sodium, le tétradécylsulfate de sodium, le tétradodécylsulfonate de sodium, l'hexadécylsulfate de sodium, l'hexadécylsulfonate de sodium, l'octadécylsulfate de sodium, l'octadécylsulfonate de sodium et le dodécylbenzylsulfonate de sodium comme constituants amphiphiles solubles dans l'eau de la solution aqueuse 2.

15. Procédé selon l'une quelconque des revendications 1 à 14, **caractérisé en ce que** les micro-espaces creux sont des pores.

16. Procédé selon l'une quelconque des revendications 1 à 15, **caractérisé en ce que** les corps présentant des systèmes de micro-espaces creux interconnectés sont constitués d'hydroxylapatite, de phosphate de tricalcium, de carbonate de calcium, de sulfate de calcium, de verre résorbable et de vitrocéramique résorbable.

17. Procédé selon l'une quelconque des revendications 1 à 14, **caractérisé en ce que** les corps présentant des micro-espaces creux interconnectés sont constitués de polymères qui sont à base d'acide L-lactique ou d'acide D-lactique et/ou d'acide glycolique et/ou d'acide 2-hydroxyéthyl-oxyacétique.

18. Procédé selon la revendication 17, **caractérisé en ce que** les corps poreux interconnectés sont présents dans la forme d'éponges, de corps en mousse, de non-tissés, de feutres, de tissus ou de tissus à maille.

19. Procédé selon l'une quelconque des revendications 1 à 18, **caractérisé en ce que** les corps sont constitués de titane, d'alliages de titane ou d'acier inoxydable.

20. Procédé selon l'une quelconque des revendications 1 à 19, **caractérisé en ce que** le revêtement antibiotique selon l'invention ne remplit pas complètement les volumes des micro-espaces creux interconnectés.

21. Procédé selon l'une quelconque des revendications 1 à 20, **caractérisé en ce que** l'on utilise du méthanol, de l'éthanol, de l'isopropanol, du N,N-diméthylformamide et/ou du diméthylsulfoxyde comme co-solvant par rapport à la solution aqueuse 1 et/ou par rapport à la solution aqueuse 2.

22. Procédé selon l'une quelconque des revendications 1 à 21, **caractérisé en ce que** l'on utilise les corps revêtus d'antiobiotiques présentant des micro-espaces creux interconnectés comme implants.

23. Procédé selon l'une quelconque des revendications 1 à 22, **caractérisé en ce que** l'on introduit la solution 2 dans les corps présentant des systèmes de micro-espaces creux interconnectés et **en ce que** l'on utilise, après l'élimination de l'eau, les corps ainsi traités présentant des systèmes de micro-espaces creux interconnectés comme matériaux d'implants, dans lesquels on introduit la solution 1 juste avant l'implantation.
